(19) Europäisches Patentamt
European Patent Office
Office européen des brevets

(11) **EP 3 349 801 B1**

(12) **EUROPEAN PATENT SPECIFICATION**

(45) Date of publication and mention
of the grant of the patent:
**17.07.2019 Bulletin 2019/29**

(51) Int Cl.:
***A61K 47/12*** *(2006.01)*      ***A61K 47/38*** *(2006.01)*
***C08B 11/00*** *(2006.01)*

(21) Application number: **16770843.7**

(22) Date of filing: **12.09.2016**

(86) International application number:
**PCT/US2016/051244**

(87) International publication number:
**WO 2017/048618 (23.03.2017 Gazette 2017/12)**

(54) **WATER-REDISPERSIBLE POLYMER POWDER**

IN WASSER REDISPERGIERBARES POLYMERPULVER

POUDRE DE POLYMÈRE REDISPERSIBLE DANS L'EAU

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **16.09.2015 US 201562219257 P**

(43) Date of publication of application:
**25.07.2018 Bulletin 2018/30**

(73) Proprietor: **Dow Global Technologies LLC
Midland, MI 48674 (US)**

(72) Inventors:
• **PETERMANN, Oliver
29699 Bomlitz (DE)**

• **FETNER, Neal J.
Midland, MI 48642 (US)**

(74) Representative: **f & e patent
Braunsberger Feld 29
51429 Bergisch Gladbach (DE)**

(56) References cited:
**EP-A1- 0 648 487          EP-A2- 0 745 383
WO-A1-2013/164121    WO-A1-2014/137779
WO-A1-2015/156922    WO-A1-2016/025568
GB-A- 2 006 217          US-A- 4 462 839**

**Description**

FIELD

This invention concerns a water-redispersible polymer powder based on an esterified cellulose ether and processes for producing the water-redispersible polymer powder.

INTRODUCTION

Esters of cellulose ethers, their uses and processes for preparing them are generally known in the art. Known methods of producing cellulose ether-esters include the reaction of a cellulose ether with an aliphatic monocarboxylic acid anhydride or a dicarboxylic acid anhydride or a combination thereof, for example as described in U.S Patent Nos. 4,226,981 and 4,365,060.

Various known esterified cellulose ethers are useful as enteric polymers for pharmaceutical dosage forms, such as methylcellulose phthalate (MCP), hydroxypropyl methylcellulose phthalate (HPMCP), methylcellulose succinate (MCS), or hydroxypropyl methylcellulose acetate succinate (HPMCAS). The esterified cellulose ethers are used for coating dosage forms, such as tablets, microparticulates or capsules. Enteric polymers protect the drug from inactivation or degradation in the acidic environment or prevent irritation of the stomach by the drug, but are dissolved in the intestinal canals to release the drug contained therein. US Patent No. 4,365,060 discloses enterosoluble capsules which are said to have excellent enterosolubility behavior.

Enteric coatings or capsules can be prepared from organic or aqueous compositions comprising esterified cellulose ethers. However, organic solvents are often not desirable for pharmaceutical or nutritional uses. On the other hand, esterified cellulose ethers only have a limited solubility in water.

The published Japanese Patent Application JP7070203A discloses a process wherein a hydroxycarboxylic acid type cellulose derivative is spread in water and pulverised by a pulveriser having a specific design to produce a cellulose derivative having a mean particle size below 7 microns, especially below 5 microns. Unfortunately, hydroxycarboxylic acid type cellulose derivatives that have been pulverized in such a manner do not form stable dispersions that can be stored over an extended time period.

European Patent Application EP 0 648 487 discloses an aqueous dispersion comprising 5 to 15 wt. % of an enteric coating base, such as HPMCAS or HPMCP. The aqueous dispersion further comprises 15 - 40 wt.% of a plasticizer, such as triethyl citrate or triacetin, and 0.1 - 10 wt.% of an anionic surfactant, such as sodium alkyl sulfate, or a sodium or potassium salt of a fatty acid, such as sodium oleate or potassium sorbate, based on the weight of HPMCAS or HPMCP.

U.S. Patent No. 4,462,839 discloses the use of cellulose acetate phthalate as enteric coating agent. The patent teaches that aqueous dispersions of cellulose acetate phthalate are not stable. It would be desirable to have the cellulose acetate phthalate particles in dry powder form than being dispersed in water. Not only would the dry powder be chemically stable but it would be considerably easier and less expensive to ship than an aqueous dispersion. However, the U.S. patent teaches that converting an aqueous dispersion to a dry powder which can be reconstituted in water to near the original particle size range is not a simple matter. When water is evaporated by any method, the particles coalesce and form a continuous film. Once the particles have coalesced there is no known way that the coalesced particles can be separated and restored to the submicron spherical size that they had while in dispersion. To solve this problem, the U.S. Patent teaches providing a freshly-prepared aqueous dispersion of cellulose acetate phthalate particles and adding a phosphate salt, such as trisodium, tripotassium or triammonium phosphate, to minimize coalescence of the particles during spray drying to produce a polymeric powder that is readily dispersible in water. Unfortunately, the freshly prepared dispersion or the dispersion obtained upon redispersing the cellulose acetate phthalate powder only contains 17 - 18 wt.-% polymer.

U.S. Patent No. 5,837,291 teaches that using enteric polymers for coating applications by dissolving the polymer in an organic solvent or applying it as aqueous latex takes a long time. Indeed when spraying coating compositions that comprise only about 15 - 18 wt. enteric coating polymer, it requires quite a long time to apply the desired amount of enteric polymer onto the dosage form. Moreover, evaporating a large amount of organic solvent or water requires much time and energy. To solve this problem, U.S. Patent No. 5,837,291 discloses a method of coating a solid dosage form with a non-solvent enteric coating agent which essentially consists of a fine powder polymeric while spraying a liquid plasticizer. However, this method requires a complex spraying device equipped with multiple nozzles. In view of this complexity, the use of aqueous dispersions for coating applications is still a method of choice.

In view of the above described disadvantages of the prior art, it is an object of the present invention to provide a water-redispersible polymer powder, wherein the polymer is an esterified cellulose ether. It is a preferred object of the present invention to provide a water-redispersible polymer powder which can be redispersed in water to provide a stable dispersion at a concentration of at least 20 wt.%. It is another preferred object of the present invention to provide a water-redispersible polymer powder which can be redispersed in water such that the particle size distribution of the redispersed polymer particles is similar or even essentially the same as the particle size distribution of the polymer particles in the dispersion from which the water-redispersible polymer powder was produced.

SUMMARY

One aspect of the present invention is a water-redispersible polymer powder, wherein the polymer is at least one esterified cellulose ether comprising (i) groups of the formula - C(O) - R - COOA or (ii) a combination of aliphatic monovalent acyl groups and groups of the formula -C(O) - R - COOA, wherein R is a divalent aliphatic or aromatic hydrocarbon group and A is hydrogen or a cation, and
the polymer powder comprises from 0.05 to 20 percent of at least one salt of a fatty acid, based on the weight of the esterified cellulose ether(s).

Another aspect of the present invention is a process for producing the above described water-redispersible polymer powder, which comprises the steps of grinding, in the presence of an aqueous diluent, at least one above-mentioned esterified cellulose ether, blending from 0.05 to 20 percent of at least one salt of a fatty acid and optionally one or more adjuvants with the esterified cellulose ether before, during or after the grinding of the esterified cellulose ether, the percentage of the fatty acid salt being based on the weight of the esterified cellulose ether, and subjecting the produced aqueous composition to spray-drying.

Yet another aspect of the present invention is a process for producing the above described water-redispersible polymer powder, which comprises the steps of melting a) at least one above-mentioned esterified cellulose ether and emulsifying the molten esterified cellulose ether in b) an aqueous diluent, adding c) from 0.05 to 20 percent of a salt of a fatty acid, based on the weight of the esterified cellulose ether, and optionally d) one or more adjuvants before, during or after the step of emulsifying the molten esterified cellulose ether in the aqueous diluent, cooling the emulsion to form an aqueous dispersion and subjecting the produced aqueous composition to spray-drying.

Yet another aspect of the present invention is a process for producing the above described water-redispersible polymer powder, which comprises the steps of providing an aqueous composition comprising a) dispersed particles of at least one above-mentioned esterified cellulose ether, and b) from 0.05 to 20 percent of at least one salt of a fatty acid, based on the weight of the dispersed esterified cellulose ether, and subjecting the aqueous composition to spray-drying.

Yet another aspect of the present invention is a process for coating a dosage form which comprises the steps of dispersing the above described water-redispersible polymer powder in water, adding a film forming aid and optional adjuvants to form an aqueous coating composition and coating a dosage form with the aqueous coating composition.

Yet another aspect of the present invention is a process for producing capsule shells which comprises the steps of dispersing the above described water-redispersible polymer powder in water, adding a film forming aid and optional adjuvants to form an aqueous composition, pre-heating molding pins to a temperature higher than that of the aqueous composition, dipping the pre-heated molding pins into the aqueous composition, forming a film on said molding pins by withdrawing said pins from said aqueous composition, and drying the film on the molding pins.

DESCRIPTION OF EMBODIMENTS

Surprisingly, a way of producing water-redispersible polymer powders has been found wherein the polymer is at least one esterified cellulose ether as described in more details below.

The water-redispersible polymer powder has great advantages, as compared to the corresponding aqueous dispersion. The dry powder is chemically stable; it is much less impacted by storage time and storage temperature than the corresponding aqueous dispersion. Also, it is not subject to microbial contamination. Moreover, it is be considerably easier and less expensive to ship the water-redispersible polymer powder than an aqueous dispersion.

Surprisingly, the water-redispersible polymer powder can be produced by spray-drying; surprisingly the particles of the water-redispersible polymer powder do not coalesce to a significant degree and do not form a continuous film. The water-redispersible polymer powder can be easily re-dispersed just by stirring or shaking.

The esterified cellulose ether which is the basis of the water-redispersible polymer powder of the present invention has a cellulose backbone having $\beta$-1,4 glycosidically bound D-glucopyranose repeating units, designated as anhydroglucose units in the context of this invention. The esterified cellulose ether preferably is an esterified alkyl cellulose, hydroxyalkyl cellulose or hydroxyalkyl alkylcellulose. This means that in the esterified cellulose ether at least a part of the hydroxyl groups of the anhydroglucose units are substituted by alkoxyl groups or hydroxyalkoxyl groups or a combination of alkoxyl and hydroxyalkoxyl groups. The hydroxyalkoxyl groups are typically hydroxymethoxyl, hydroxyethoxyl and/or hydroxypropoxyl groups. Hydroxyethoxyl and/or hydroxypropoxyl groups are preferred. Typically one or two kinds of hydroxyalkoxyl groups are present in the esterified cellulose ether. Preferably a single kind of hydroxyalkoxyl group, more preferably hydroxypropoxyl, is present. The alkoxyl groups are typically methoxyl, ethoxyl and/or propoxyl groups. Methoxyl groups are preferred. Illustrative of the above-defined esterified cellulose ethers are esterified alkylcelluloses, such as esterified methylcelluloses, ethylcelluloses, and propylcelluloses; esterified hydroxyalkylcelluloses, such as esterified hydroxyethylcelluloses, hydroxypropylcelluloses, and hydroxybutylcelluloses; and esterified hydroxyalkyl alkylcelluloses, such as esterified hydroxyethyl methylcelluloses, hydroxymethyl ethylcelluloses, ethyl hydroxyethylcelluloses, hydroxypropyl methylcelluloses, hydroxypropyl ethylcelluloses, hydroxybutyl methylcelluloses, and hydroxybutyl ethylcelluloses; and those having two or more hydroxyalkyl groups, such as esterified hydroxyethylhydroxypropyl methylcelluloses. Most preferably, the esterified cellulose ether is an esterified hydroxyalkyl methylcellulose, such as an esterified hydroxypropyl methylcellulose.

The degree of the substitution of hydroxyl groups of the anhydroglucose units by hydroxyalkoxyl groups is expressed by the molar substitution of hydroxyalkoxyl groups, the MS(hydroxyalkoxyl). The MS(hydroxyalkoxyl) is the average number of moles of hydroxyalkoxyl groups per anhydroglucose unit in the esterified cellulose ether. It is to be understood that during the hydroxyalkylation reaction the hydroxyl group of a hydroxyalkoxyl group bound to the cellulose backbone can be further etherified by an alkylating agent, e.g. a methylating agent, and/or a hydroxyalkylating agent. Multiple subsequent hydroxyalkylation etherification reactions with respect to the same carbon atom position of an anhydroglucose unit yields a side chain, wherein multiple hydroxyalkoxyl groups are covalently bound to each other by ether bonds, each side chain as a whole forming a hydroxyalkoxyl substituent to the cellulose backbone.

The term "hydroxyalkoxyl groups" thus has to be interpreted in the context of the MS(hydroxyalkoxyl) as referring to the hydroxyalkoxyl groups as the constituting units of hydroxyalkoxyl substituents, which either comprise a single hydroxyalkoxyl group or a side chain as outlined above, wherein two or more hydroxyalkoxyl units are covalently bound to each other by ether bonding. Within this definition it is not important whether the terminal hydroxyl group of a hydroxyalkoxyl substituent is further alkylated or not; both alkylated and non-alkylated hydroxyalkoxyl substituents are included for the determination of MS(hydroxyalkoxyl). The esterified cellulose ether generally has a molar substitution of hydroxyalkoxyl groups of at least 0.05, preferably at least 0.08, more preferably at least 0.12, and most preferably at least 0.15. The degree of molar substitution is generally not more than 1.00, preferably not more than 0.90, more preferably not more than 0.70, and most preferably not more than 0.50.

The average number of hydroxyl groups substituted by alkoxyl groups, such as methoxyl groups, per anhydroglucose unit, is designated as the degree of substitution of alkoxyl groups, DS(alkoxyl). In the above-given definition of DS, the term "hydroxyl groups substituted by alkoxyl groups" is to be construed within the present invention to include not only alkylated hydroxyl groups directly bound to the carbon atoms of the cellulose backbone, but also alkylated hydroxyl groups of hydroxyalkoxyl substituents bound to the cellulose backbone. The esterified cellulose ethers preferably have a DS(alkoxyl) of at least 1.0, more preferably at least 1.1, even more preferably at least 1.2, most preferably at least 1.4, and particularly at least 1.6. The DS(alkoxyl) is preferably not more than 2.5, more preferably not more than 2.4, even more preferably not more than 2.2, and most preferably not more than 2.05.

Most preferably the esterified cellulose ether is an esterified hydroxypropyl methylcellulose having a DS(methoxyl) within the ranges indicated above for DS(alkoxyl) and an MS(hydroxypropoxyl) within the ranges indicated above for MS(hydroxyalkoxyl).

The esterified cellulose ether utilized in the present invention has (i) groups of the formula -C(O) - R - COOA or (ii) a combination of aliphatic monovalent acyl groups and groups of the formula -C(O) - R - COOA, wherein R is a divalent aliphatic or aromatic hydrocarbon group and A is hydrogen or a cation. The cation preferably is an ammonium cation, such as $NH_4^+$ or an alkali metal ion, such as the sodium or potassium ion, more preferably the sodium ion. Most preferably, A is hydrogen.

The aliphatic monovalent acyl groups are preferably selected from the group consisting of acetyl, propionyl, and butyryl, such as n-butyryl or i-butyryl.

Preferred groups of the formula - C(O) - R - COOA are - C(O) - $CH_2$ - $CH_2$ - COOA, such as - C(O) - $CH_2$ - $CH_2$ - COOH or - C(O) - $CH_2$ - $CH_2$ - $COO^-Na^+$, - C(O) - CH = CH - COOA, such as - C(O) - CH = CH - COOH or - C(O) - CH = CH - $COO^-Na^+$, or
- C(O) - $C_6H_4$ - COOA, such as - C(O) - $C_6H_4$ - COOH or - C(O) - $C_6H_4$ - $COO^-Na^+$. In the groups of formula - C(O) - $C_6H_4$ - COOA the carbonyl group and the carboxylic group are preferably arranged in ortho-positions.

In the esterified cellulose ether the degree of neutralization of the groups - C(O) - R - COOA is preferably not more than 0.4, more preferably not more than 0.3, even more preferably not more than 0.2, most preferably not more than 0.1, and particularly not more than 0.05 or even not more than 0.01. The degree of neutralization can even be essentially zero or only slightly above it, e.g. up to $10^{-3}$ or even only up to $10^{-4}$. The term "degree of neutralization" as used herein defines the ratio of deprotonated carboxylic groups over the sum of deprotonated and protonated carboxylic groups, i.e.,

$$\text{Degree of neutralization} = [\text{--C(O)--R-- COO}^-] / [\text{--C(O)--R--COO}^- + \text{--C(O)--R--COOH}].$$

Preferred esterified cellulose ethers are

i) HPMCXY, wherein HPMC is hydroxypropyl methyl cellulose, X is A (acetate), or X is B (butyrate) or X is Pr (propionate) and Y is S (succinate), or Y is P (phthalate) or Y is M (maleate), such as hydroxypropyl methyl cellulose acetate phthalate (HPMCAP), hydroxypropyl methyl cellulose acetate maleate (HPMCAM), or hydroxypropyl methylcellulose acetate succinate (HPMCAS), or

ii) hydroxypropyl methyl cellulose phthalate (HPMCP), hydroxypropyl cellulose acetate succinate (HPCAS), hydroxybutyl methyl cellulose propionate succinate (HBMCPrS), hydroxyethyl hydroxypropyl cellulose propionate succinate (HEHPCPrS); and methyl cellulose acetate succinate (MCAS).

Hydroxypropyl methylcellulose acetate succinate (HPMCAS) is the most preferred esterified cellulose ether.

The esterified cellulose ethers generally have a degree of substitution of aliphatic monovalent acyl groups, such as acetyl, propionyl, or butyryl groups, of not more than 1.75, preferably not more than 1.50, more preferably not more than 1.25, and most preferably not more than 1.00, or even not more than 0.65. The degree of substitution of aliphatic monovalent acyl groups can be zero, but preferably it is at least 0.05, more preferably at least 0.10, and most preferably at least 0.20.

The esterified cellulose ethers generally have a degree of substitution of groups of formula -C(O) - R - COOA, such as succinoyl, of at least 0.05, preferably at least 0.10. The degree of substitution of groups of formula -C(O) - R - COOA generally is up to 1.6, preferably up to 1.30, more preferably up to 1.00, and most preferably up to 0.70 or even up to 0.60.

The sum of i) the degree of substitution of aliphatic monovalent acyl groups and ii) the degree of substitution of groups of formula -C(O) - R - COOA is generally at least 0.05, preferably at least 0.10, more preferably at least 0.20, most preferably at least 0.30, and particularly at least 0.40. The mentioned sum is generally no more than 2.0, preferably no more than 1.4, more preferably no more than 1.15, most preferably no more than 1.10 and particularly no more than 1.00.

The content of the acetate and succinate ester groups is determined according to "Hypromellose Acetate Succinate", United States Pharmacopeia and National Formulary, NF 29, pp. 1548-1550. Reported values are corrected for volatiles (determined as described in section "loss on drying" in the above HPMCAS monograph). The method may be used in analogue manner to determine the content of propionyl, butyryl, phthalyl and other ester groups.

The content of ether groups in the esterified cellulose ether is determined in the same manner as described for "Hypromellose", United States Pharmacopeia and National Formulary, USP 35, pp 3467-3469.

The contents of ether and ester groups obtained by the above analyses are converted to DS and MS values of individual substituents according to the formulas below. The formulas may be used in analogue manner to determine the DS and MS of substituents of other cellulose ether esters.

$$
\begin{aligned}
\% \text{ cellulose backbone} \\
= 100 - \left( \%\text{MeO} * \frac{M(OCH_3) - M(OH)}{M(OCH_3)} \right) \\
- \left( \%\text{HPO} * \frac{M(OCH_2CH(OH)CH_3) - M(OH)}{M(OCH_2CH(OH)CH_3)} \right) \\
- \left( \%\text{Acetyl} * \frac{M(COCH_3) - M(H)}{M(COCH_3)} \right) \\
- \left( \%\text{Succinoyl} * \frac{M(COC_2H_4COOH) - M(H)}{M(COC_2H_4COOH)} \right)
\end{aligned}
$$

$$
DS(Me) = \frac{\dfrac{\%\text{MeO}}{M(OCH_3)}}{\dfrac{\%\text{cellulose backbone}}{M(AGU)}}
\qquad
MS(HP) = \frac{\dfrac{\%\text{HPO}}{M(HPO)}}{\dfrac{\%\text{cellulose backbone}}{M(AGU)}}
$$

$$
DS(Acetyl) = \frac{\dfrac{\%\text{Acetyl}}{M(Acetyl)}}{\dfrac{\%\text{cellulose backbone}}{M(AGU)}}
\qquad
DS(Succinoyl) = \frac{\dfrac{\%\text{Succinoyl}}{M(Succinoyl)}}{\dfrac{\%\text{cellulose backbone}}{M(AGU)}}
$$

$$M(MeO) = M(OCH_3) = 31.03\,Da \qquad M(HPO) = M(OCH_2CH(OH)CH_3) = 75.09\,Da$$

$$\begin{aligned} M(Acetyl) &= M(COCH_3) \\ &= 43.04\,Da \end{aligned} \qquad \begin{aligned} M(Succinoyl) &= M(COC_2H_4COOH) \\ &= 101.08\,Da \end{aligned}$$

$$M(AGU) = 162.14\,Da \qquad M(OH) = 17.008\,Da \qquad M(H) = 1.008\,Da$$

By convention, the weight percent is an average weight percentage based on the total weight of the cellulose repeat unit, including all substituents. The content of the methoxyl group is reported based on the mass of the methoxyl group (i.e., $-OCH_3$). The content of the hydroxyalkoxyl group is reported based on the mass of the hydroxyalkoxyl group (i.e., -O-alkylene-OH); such as hydroxypropoxyl (i.e., $-O-CH_2CH(CH_3)-OH$). The content of the aliphatic monovalent acyl group is reported based on the mass of $-C(O)- R_1$ wherein $R_1$ is a monovalent aliphatic group, such as acetyl ($-C(O)-CH_3$). The content of the group of formula -C(O) - R - COOH is reported based on the mass of this group, such as the mass of succinoyl groups (i.e., - C(O) - $CH_2$ - $CH_2$ - COOH).

The esterified cellulose ether generally has a viscosity of at least 1.2 mPa·s, preferably least 1.8 mPa·s, and more preferably least 2.4 mPa·s, and generally no more than 200 mPa·s, preferably no more than 100 mPa·s, more preferably no more than 50 mPa·s, and most preferably no more than 30 mPa·s, measured as a 2.0 weight percent solution of the esterified cellulose ether in 0.43 wt% aqueous NaOH at 20°C according to "Hypromellose Acetate Succinate, United States Pharmacopia and National Formulary, NF 29, pp. 1548-1550".

The water-redispersible polymer powder further comprises from 0.05 to 20 percent of at least one salt of a fatty acid, based on the weight of the dispersed esterified cellulose ether. The total amount of the salt(s) of a fatty acid preferably is at least 0.1 percent, more preferably at least 0.3 percent, even more preferably at least 0.5 percent, most preferably at least 0.8 percent, and particularly at least 1.0 percent, based on the total weight of the esterified cellulose ether(s). The total amount of the salt(s) of a fatty acid preferably is up to 15 percent, more preferably up to 12 percent, even more preferably up to 10 percent or 8 percent, and most preferably up to 6.0 percent, or even only up to 5.0 percent, based on the total weight of the esterified cellulose ether(s).

Preferred fatty acid salts are ammonium, alkali metal or alkaline earth metal salts. A preferred ammonium ion is $NH_4^+$. Preferred alkali metal ions are the sodium or potassium ions. A preferred alkaline earth metal ion is the calcium ion. The fatty acids can be saturated or unsaturated. Exemplary of saturated fatty acids are caprylic acid, capric acid, lauric acid, myristic acid, palmitic acid, stearic acid, arachidic acid, behenic acid, lignoceric acid and cerotic acid. The unsaturated fatty acids can be mono-, di- or tri- unsaturated fatty acids, mono-unsaturated and di- unsaturated fatty acids being preferred. Exemplary of mono-unsaturated fatty acids are myristoleic acid, palmitoleic acid, sapienic acid, oleic acid, elaidic acid and vaccenic acid. Exemplary of di-unsaturated fatty acids are linoleic acid and linoelaidic acid. Ammonium, alkali metal and alkaline earth metal salts of stearic acid or oleic acid are most preferred, particularly those salts mentioned above.

Optionally the water-redispersible polymer powder also comprises from 0.01 to 10 percent of an anionic surfactant comprising a sulfate or sulfonate group, based on the weight of the esterified cellulose ether. The total amount of anionic surfactant(s) comprising a sulfate or sulfonate group, if present, preferably is at least 0.02 percent, more preferably at least 0.05 percent, most preferably at least 0.1 percent, and particularly at least 0.2 percent, based on the total weight of the esterified cellulose ether(s). The total amount of the anionic surfactant(s) comprising a sulfate or sulfonate group preferably is up to 7 percent, more preferably up to 5 percent, most preferably up to 3 percent, and particularly only up to 1.5 percent, based on the total weight of the esterified cellulose ether(s).

Preferred are ammonium, alkali metal or alkaline earth metal salts of the anionic surfactant comprising a sulfate or sulfonate group. The anionic surfactant can have one or more, preferably one or two, sulfate or sulfonate groups. A preferred ammonium ion is $NH_4^+$. Preferred alkali metal ions are the sodium or potassium ions. A preferred alkaline earth metal ion is the calcium ion. Preferably the anionic surfactant comprises one or more, preferably one or two, alkyl, alkenyl, alkinyl or cycloalkyl groups. Preferably the alkyl, alkenyl, alkinyl or cycloalkyl groups each independently comprise from 4 to 12 carbon atoms, more preferably from 6 to 8 carbon atoms. The total number of carbon atoms in the alkyl, alkenyl, alkinyl or cycloalkyl group(s) in the surfactant is preferably 8 to 24, more preferably 12 to 16. These groups can be branched or linear. Preferred anionic surfactants comprising a sulfate or sulfonate group are alkyl sulfate salts, such as ammonium lauryl sulfate, potassium lauryl sulfate, sodium dodecyl sulfate (SDS) or sodium laureth sulfate (SLES); or alkyl benzene sulphonates, such as sodium dodecylbenzenesulfonate, or alkyldiphenyloxide disulfonate salts, such as sodium dodecyl diphenyl ether disulfonate. More preferred anionic surfactants comprising a sulfate or sulfonate group are dialkyl, dialkenyl, dialkinyl or dicycloalkyl sulfosuccinates. Most preferred is a dialkyl sulfosuccinate, particularly dioctyl sodium sulfosuccinate (IUPAC name sodium 1,4-bis(2-ethylhexoxy)-1,4-dioxobutane-2-sulfonate), dioctyl potassium sulfosuccinate, or dioctyl calcium sulfosuccinate.

The median particle size, Dn50, of the water-redispersible polymer powder is typically up to 5 micrometers, more typically up to 4 micrometers, and even more typically up to 3 micrometers. The median particle size, Dn50, of the water-redispersible polymer powder is typically 0.3 micrometers or more, more typically 1.0 micrometers or more, and most typically 1.5 micrometers or more. The median particle size Dn50 is the diameter where 50 number percent of the particles have a smaller equivalent diameter and 50 number percent have a larger equivalent diameter. Typically Dn90 is 1.0 micrometers or more, more typically 2.0 micrometers or more, and most typically 4.0 micrometers or more; and typically up to 12 micrometers, more typically up to 10 micrometers, and most typically up to 7 micrometers. Dn90 is the diameter where 90 number percent of the particles have a smaller equivalent diameter and the other 10 number percent have a larger equivalent diameter. The equivalent particle diameter is the diameter of a sphere having the same volume as the volume of a given particle. The mean particle diameter is typically 0.5 micrometers or more, more typically 1.0 micrometers or more, and most typically 2.0 micrometers or more; and typically up to 8 micrometers, more typically up to 6 micrometers, and most typically even only up to 4 micrometers. The mean particle diameter is the number mean. These particle sizes relate to the sizes of the esterified cellulose ether particles after redispersion in water. After redispersion in water the particle sizes are measured by laser diffraction particle size analysis, e.g., using a Beckman Coulter laser diffraction particle size analyzer which is commercially available from Beckman Coulter, California.

The water-redispersible polymer powder can be prepared by providing an aqueous composition which comprises a) dispersed particles of at least one esterified cellulose ether as described above and b) from 0.05 to 20 percent of at least one salt of a fatty acid, based on the weight of the dispersed esterified cellulose ether, and subjecting the aqueous composition to spray-drying.

The aqueous composition comprises an aqueous diluent. The aqueous diluent is water, optionally mixed with a minor amount of an organic solvent. The aqueous diluent preferably consists of 50 - 100 weight percent, more preferably 65 - 100 weight percent, and most preferably 75 - 100 weight percent of water and preferably 0 - 50 weight percent, more preferably 0 - 35 weight percent, and most preferably 0 - 25 weight percent of an organic solvent, based on the total weight of water and the organic solvent. Useful organic solvents are polar organic solvents having one or more heteroatoms, such as oxygen, nitrogen or halogen like chlorine. More preferred organic solvents are alcohols, for example multifunctional alcohols, such as glycerol, or preferably monofunctional alcohols, such as methanol, ethanol, isopropanol or n-propanol; ethers, such as tetrahydrofuran, ketones, such as acetone; methyl ethyl ketone, or methyl isobutyl ketone; acetates, such as ethyl acetate; halogenated hydrocarbons, such as methylene chloride; or nitriles, such as acetonitrile. Preferably the aqueous composition comprises water alone as aqueous diluent. The amount of the aqueous diluent is typically at least 50 percent, more typically at least 55 percent, based on the total weight of the aqueous composition. The amount of the aqueous diluent is typically no more than 79 percent, more typically no more than 75 percent, based on the total weight of the aqueous composition.

The aqueous composition which comprises a) dispersed particles of at least one esterified cellulose ether and b) from 0.05 to 20 percent of at least one salt of a fatty acid, based on the weight of the dispersed esterified cellulose ether, can be prepared by various methods as described below. However, preparing an aqueous composition by simply physically blending an esterified cellulose ether, a salt of a fatty acid and an aqueous diluent at room temperature is not suitable for preparing a stable dispersion.

In one embodiment the process for producing the aqueous composition comprises the steps of grinding, in the presence of an aqueous diluent, at least one esterified cellulose ether, blending a salt of a fatty acid, optionally an anionic surfactant comprising a sulfate or sulfonate group and optionally one or more further adjuvants with the esterified cellulose ether before, during or after the grinding of the esterified cellulose ether, and choosing the amounts of aqueous diluent, esterified cellulose ether, salt of a fatty acid, optionally anionic surfactant comprising a sulfate or sulfonate group and optionally one or more adjuvants such that the produced aqueous composition comprises a) generally at least 20 percent, based on the total weight of the aqueous composition, of the dispersed esterified cellulose ether, b) from 0.05 to 20 percent of the salt of a fatty acid, and optionally c) from 0.01 to 10 percent of the anionic surfactant comprising a sulfate or sulfonate group, the percentages of components b) and c) being based on the weight of the dispersed esterified cellulose ether. The salt of a fatty acid, optionally the anionic surfactant comprising a sulfate or sulfonate group and optional adjuvants are preferably added before or during the grinding of the esterified cellulose ether in the aqueous diluent. A salt of a fatty acid, an anionic surfactant comprising a sulfate or sulfonate group and optional adjuvants can also be added after the grinding of the esterified cellulose ether, but generally at least 50 percent of the salt of a fatty acid that is used for preparing the aqueous composition is added before or during the grinding of the esterified cellulose ether.

Any grinding device suitable for grinding esterified cellulose ethers in the presence of an aqueous diluent to a median particle size, Dn50, of up to 5 micrometers, more typically up to 4 micrometers, and even more typically up to 3 micrometers can be used. Preferred grinding devices are wet grinding units such as media mills or bead mills. The grinding is typically conducted at a temperature of at least 10 °C, preferably of at least 30 °C, and typically at a temperature of up to 55 °C, more typically up to 50 °C. The most preferred grinding temperature is of from 37 to 55 °C, particularly from 40 to 55 °C. Grinding is generally conducted for a sufficient time period to achieve an above-mentioned median particle size, Dn50, of the dispersed esterified cellulose ether particles. The grinding period is typically from 60 to 180 minutes.

In another embodiment the process for producing the aqueous composition comprises the steps of melting at least one esterified cellulose ether and emulsifying the molten esterified cellulose ether in an aqueous diluent, adding a salt of a fatty acid, optionally an anionic surfactant comprising a sulfate or sulfonate group and optionally one or more adjuvants before, during or after the step of emulsifying the molten esterified cellulose ether in the aqueous diluent, choosing the amounts of aqueous diluent, esterified cellulose ether, salt of a fatty acid, optionally anionic surfactant comprising a sulfate or sulfonate group and optionally one or more adjuvants such that the produced aqueous composition comprises a) generally at least 20 percent, based on the total weight of the aqueous composition, of the dispersed esterified cellulose ether, b) from 0.05 to 20 percent of the salt of a fatty acid, and optionally c) from 0.01 to 10 percent of the anionic surfactant comprising a sulfate or sulfonate group, the percentages of components b) and c) being based on the weight of the dispersed esterified cellulose ether, and cooling the emulsion to form an aqueous dispersion. This embodiment of the process is preferably conducted in an extruder. Alternatively, a pressurized batch kneader can be used for conducting this embodiment of the invention. The general process conditions and equipment which are useful to perform the process are disclosed in U.S. Patent Nos. 5,539,021 and 7,763,676.

After the production of the aqueous composition as described above, the dispersed esterified cellulose ether particles generally have a median particle size, Dn50, of up to 5 micrometers, more typically up to 4 micrometers, and even more typically up to 3 micrometers. The median particle size, Dn50, of the dispersed esterified cellulose ether particles is typically 0.3 micrometers or more, more typically 1.0 micrometers or more, and most typically 1.5 micrometers or more. The median particle size, Dn50, is the diameter where 50 number percent of the particles have a smaller equivalent diameter and 50 number percent have a larger equivalent diameter. Typically Dn90 is 1.0 micrometers or more, more typically 2.0 micrometers or more, and most typically 4.0 micrometers or more; and typically up to 12 micrometers, more typically up to 10 micrometers, and most typically up to 7 micrometers. Dn90 is the diameter where 90 number percent of the particles have a smaller equivalent diameter and the other 10 number percent have a larger equivalent diameter. The equivalent particle diameter is the diameter of a sphere having the same volume as the volume of a given particle. The mean particle diameter is typically 0.5 micrometers or more, more typically 1.0 micrometers or more, and most typically 2.0 micrometers or more; and typically up to 8 micrometers, more typically up to 6 micrometers, and most typically even only up to 4 micrometers. The mean particle diameter is the number mean.

Surprisingly, by the above-mentioned processes aqueous compositions can be prepared which comprise at least 20 percent, preferably at least 25 percent, more preferably at least 30 percent, and most preferably even at least 35 percent of the esterified cellulose ether(s) in dispersed state in the aqueous composition. The aqueous composition generally comprises up to 40 percent or in some cases even up to 43 percent of the esterified cellulose ether(s) in dispersed state in the aqueous composition. These percentages are based on the total weight of the composition. In spite of the very high concentration of dispersed esterified cellulose ether(s), the aqueous composition has a reasonably low viscosity which allows easy spray-drying. The apparent viscosity of the aqueous composition is generally 50 mPa·s or more, typically 100 mPa·s or more, measured at 20 ° C. The apparent viscosity of the aqueous composition is generally no more than 5000 mPa·s, typically no more than 4000 mPa·s, measured at 20 ° C.

There is a significant processing window between the preparation of the aqueous composition comprising dispersed esterified cellulose ether particles and spray-drying. The aqueous composition can be stored for at least 2 weeks, in its preferred embodiments even more than 3 months, and in the most preferred embodiments even more than 6 months, without losing its ability to flow under its own weight if it do not comprise a film forming aid.

Spray-drying devices are known in the art. Preferred spray-drying devices are conventional spray-dryers like Mini Spray Dryer B-290 from Büchi Labortechnik AG, Mobile Minor or Production Minor from GEA Group Aktiengesellschaft.. Preferably a gas, such as air or nitrogen is used for spray-drying. The temperature in the spray-drying device preferably is from 100 to 300 °C, more preferably from 150 to 220 °C. The outlet temperature of the spray-drying device, i.e., the temperature in the spray nozzle(s) typically is from 50 to 200 °C, more typically from 70 to 110 °C. During spray-drying, the individual polymer particles may become grouped together in small aggregates, but the particles are readily redispersible in water at a later time.

It is very surprising that after spray-drying essentially the same particle size distribution of the water-redispersible polymer powder is achieved as in the aqueous dispersion before spray-drying. Without wanting to be bound by the theory, it is believed that upon spray-drying the salt of a fatty acid prevents or minimizes coalescence of the esterified cellulose ether particles.

Moreover, it is very surprising that the above-mentioned aqueous composition comprising the dispersed esterified cellulose ether particles can be spray-dried at all without substantial and repeated clogging of the spray-drying nozzles, which would result in operational failures. Aqueous dispersions of esterified cellulose ethers typically exhibit a phase transition temperature, which results in a drastic gel-like viscosity increase of the aqueous dispersion. When the esterified cellulose ether is hydroxypropyl methyl cellulose acetate succinate (HPMCAS), the phase transition is typically at about 30 to 40°C. It is very surprising that no substantial and repeated clogging of the spray-drying nozzles is experienced although the outlet temperature of the spray-dryer is substantially above that of the phase transition of HPMCAS.

Upon spray-drying of the aqueous composition as described further above a water-redispersible polymer powder is obtained which is chemically more stable and much less impacted by storage time and storage temperature than the corresponding aqueous dispersion. Also, the redispersible polymer powder is not subject to microbial contamination. Moreover, it is be considerably easier and less expensive to ship the water-redispersible polymer powder than the aqueous dispersion.

Shortly before its use, the water-redispersible polymer powder can easily be re-dispersed in water, e.g., by simple stirring or shaking. Cooling or heating is typically not needed. The water typically can have room temperature. Usually only few minutes, e.g. 1 - 5 minutes, are sufficient to re-disperse the water-redispersible polymer powder in water. Surprisingly, upon re-dispersion of the polymer powder in water, aqueous dispersions can be prepared which comprise at least 20 percent, preferably at least 25 percent, more preferably at least 30 percent, and most preferably even at least 35 percent of the esterified cellulose ether(s). The aqueous dispersions generally comprise up to 40 percent or in some cases even up to 43 percent of the esterified cellulose ether(s) in dispersed state. These percentages are based on the total weight of the aqueous dispersion. Even after storage for several days no particle agglomeration and no sediment is observed. The particle size distribution of the redispersed polymer particles in water is similar or even essentially the same as the particle size distribution of the polymer particles in the original dispersion from which the water-redispersible polymer powder was produced.

In another aspect of the invention the water-redispersible polymer powder of the present invention is used for coating dosage forms, such as tablets, granules, pellets, caplets, lozenges, suppositories, pessaries or implantable dosage forms, to form a coated composition. The process for coating a dosage form comprising the step of dispersing the water-redispersible polymer powder in water as described above, adding a film forming aid and optional adjuvants to form an aqueous coating composition and coating a dosage form with the aqueous coating composition. If the aqueous composition comprises an active ingredient, such as a drug, drug layering can be achieved, i.e., the dosage form and the coating may comprise different active ingredients for different end-uses and/or having different release kinetics. The coating can be carried out in a known manner, for example by known dipping or spraying processes.

In yet another aspect of the invention the water-redispersible polymer powder of the present invention is used for producing capsule shells. The process for producing capsule shells comprises the steps of dispersing the water-redispersible polymer powder in water as described above, adding a film forming aid and optional adjuvants to form an aqueous composition, pre-heating molding pins to a temperature higher than that of the aqueous composition, dipping the pre-heated molding pins into the aqueous composition, forming a film on said molding pins by withdrawing said pins from said aqueous composition, and drying the film on the molding pins. The general process conditions and equipment which may be used to prepare capsules shells are described in International Patent Application Nos. WO 2013/164122 and WO 2013/164121, the disclosures of which are incorporated herein by reference.

The term "film forming aid" comprises one or more plasticizers conventionally used in the manufacture of coatings or capsule shells, notably hard capsule shells, to ensure the formation of self-supported cohesive films and avoid capsule brittleness, and/or one or more viscosity enhancers at elevated temperature, i.e. natural as well as synthetic substances conventionally used to optimize aqueous compositions for coating purposes or the dip molding manufacture of hard capsule shells.

Film forming aids that display plasticizing properties include: phthalic esters, such as dimethyl-, diethyl-, and diisopropyl-phthalate; citric esters, such as triethyl-, tributyl-, acetyltriethyl- and acetyltributyl-citrate; phosphoric esters, such as triethyl-, tricresyl, and triphenyl-phosphate; alkyl lactate; glycol esters; glycerol and glycerol esters, such as glycerol triacetate also known as triacetine; sucrose esters; oils and fatty acid esters; butyl stearate; dibutyl sebacate; dibutyl tartrate; diisobutyl adipate, tributyrin; propylene glycol; and mixtures thereof.

In one embodiment, film forming aids are cellulose ethers, such as carboxy methylcellulose, hydroxypropyl cellulose, ethyl cellulose, methylcellulose, hydroxypropylmethylcellulose (HPMC), e.g. HPMC types 2910, 2906 and/or 2208 as defined in USP30- NF25; gelatin, pullulan, non enteric starch derivatives, such as hydroxypropyl starch; polyvinyl acetate derivatives (PVAP); sorbitan monoesters; sorbitan polyoxyethylene esters; fatty acid esters; glycerol polyethylene, glycol ricinoleate; macrogolglycerides; triethyl citrate (TEC); acetyl trialkyl citrate; glycerol triacetate (triacetine); talc; and mixtures thereof.

The amount of the film forming aid preferably is least 5 percent, more preferably at least 10 percent, even more preferably at least 13 percent, and most preferably at least 15 percent, based on the weight of the esterified cellulose ether. The amount of the film forming aid is generally up to 30 percent, preferably up to 25 percent, even more preferably up to 22 percent, and most preferably up to 20 percent, based on the weight of the esterified cellulose ether.

The aqueous composition used for coating dosage forms or for producing capsules may further comprise optional ingredients, for example active ingredients, such as fertilizers, herbicides, pesticides, or biologically active ingredients, such as vitamins, herbals or mineral supplements or drugs; or adjuvants such as coloring agents, pigments, opacifiers, flavor or taste improvers, antioxidants, or any combination thereof. Optional ingredients are preferably pharmaceutically acceptable. The amount of these optional ingredients is typically from 0 to 50 percent of the total weight of the ingredients of the aqueous composition excluding the aqueous diluent. Typically the amount is 1 percent or more, more typically 5 percent or more; and typically up to 40 percent, more typically up to 20 percent of the total weight of the ingredients of the aqueous composition excluding the aqueous diluent.

The water-redispersible polymer powder of the present invention is particularly useful for coating dosage forms or for the formation of capsules shells for enteric use, i.e., coatings or capsules shells that are dissolved in the intestinal canals to release the active ingredient like a drug contained in the dosage form or in the capsules.

EXAMPLES

Unless otherwise mentioned, all parts and percentages are by weight. In the Examples the following test procedures are used.

Viscosity of Hydroxypropyl Methyl Cellulose Acetate Succinate (HPMCAS)

A 2.0 % by weight solution of the HPMCAS in 0.43 wt.% aqueous NaOH was prepared as described in "Hypromellose Acetate Succinate, United States Pharmacopia and National Formulary, NF 29, pp. 1548-1550, followed by an Ubbelohde viscosity measurement at 20 °C according to DIN 51562-1:1999-01 (January 1999).

Content of ether and ester groups of HPMCAS

The content of ether groups in HPMCAS was determined in the same manner as described for "Hypromellose", United States Pharmacopeia and National Formulary, USP 35, pp 3467-3469. The ester substitution with acetyl groups ($-CO-CH_3$) and the ester substitution with succinoyl groups ($-CO-CH_2-CH_2-COOH$) were determined according to Hypromellose Acetate Succinate, United States Pharmacopia and National Formulary, NF 29, pp. 1548-1550". Reported values for ester substitution were corrected for volatiles (determined as described in section "loss on drying" in the above HPMCAS monograph).

HPMCAS particle size measurement in the aqueous dispersion

To measure particle sizes 1-2 g of the aqueous HPMCAS dispersion that had been produced as described below was diluted in 20 ml of purified water. The particle size in the diluted dispersion was measured by laser diffraction particle size analysis using a Beckman Coulter LS 13 320 laser diffraction particle size analyzer which is commercially available from Beckman Coulter, California. The Universal Liquid Module (ULM) with a Fraunhofer optical model, a Polarization Intensity Differential Scattering (PIDS) system and a sonication control unit were used. In the sonication control unit the HPMCAS dispersion was subjected to ultrasonic treatment for a time period of up to 120 seconds during the HPMCAS addition (about 30 seconds) and particle size measurement (about 90 seconds). The number particle size distribution of the particles is listed in Table 1 below, specifically the number mean, and the values Dn10, Dn25, Dn50, Dn75 and Dn90. Dnx is the particle diameter where x number percent of the powder particles have a smaller equivalent diameter and the other (100 - x) number percent have a larger equivalent diameter. The equivalent particle diameter is the diameter of a sphere having the same volume as the volume of a given particle.

Particle size measurement of the water redispersible HPMCAS polymer powder after re-dispersion in water

Water was added to the water-redispersible HPMCAS polymer powder that had been prepared by spray-drying a HPMCAS dispersion as described in the Examples below. The amount of water was chosen to provide the same HPMCAS concentration as in the original HPMCAS dispersions from which the water-redispersible polymer HPMCAS powder had been produced. The concentrations are listed in Table 1 below.

The addition of water was followed by shaking for 1 min. to disperse the HPMCAS powder in water. The particle size of the water-redispersible HPMCAS polymer powder after re-dispersion in water was measured by laser diffraction particle size analysis using a Beckman Coulter LS 13 320 laser diffraction particle size analyzer as described above for the aqueous dispersion. As in the procedure further above, 1-2 g of the aqueous HPMCAS re-dispersion was diluted in 20 ml of purified water to measure the HPMCAS particle sizes.

Apparent viscosity of the aqueous HPMCAS dispersion of Examples 1 and 2

The apparent viscosity of the aqueous dispersion comprising HPMCAS was measured at various temperatures according to a temperature sweep experiment performed with a Anton Paar MCR 301 rheometer with a CC-27 cup geometry and a 4-blade vane geometry ST26-4V-20 over a temperature range of 10 to 50 °C with a heating rate of 0.5 °C / min and a constant speed of the vane geometry of 40 rpm and a measurement point duration of 0.25 min. Prior to this temperature sweep testing the material was treated for 30 min at 10 °C at 250 rpm. A sample volume of 20 ml was used for these measurements. The samples had been stored at room temperature prior to the viscosity measurement.

Determination of solids content of the aqueous HPMCAS dispersion

The solids content was determined using a moisture balance (Mettler Toledo Advanced Moisture Analyzer, Model HB43-S). Instrument settings were as follows: 3 g dispersion using the Rapid drying program with a temperature set point of 120 °C (40% overshoot for first 3 minutes) with switch-off criteria 5 (less than 1 mg weight change over 140 seconds). Upon drying to remove water, the residual solids content (including all additives) was weighed.

HPMCAS used in Example 1

HPMCAS was used that had 23.0 % methoxyl groups ($DS_{methoxyl}$ = 1.89), 7.3 % hydroxypropoxyl groups ($MS_{hydroxypropoxyl}$ = 0.25), 9.1 % acetyl groups ($DS_{acetyl}$ = 0.54), 11.6% succinoyl groups ($DS_{succinoyl}$ = 0.29), and a viscosity of 2.9 mPa·s, measured as a 2.0 % by weight solution of the HPMCAS in 0.43 wt.% aqueous NaOH.

HPMCAS used in Example 2 and 3

HPMCAS was used that had 23.2 % methoxyl groups ($DS_{methoxyl}$ = 1,90), 7.4 % hydroxypropoxyl groups ($MS_{hydroxypropoxyl}$ = 0.25), 9.4 % acetyl groups ($DS_{acetyl}$ = 0.56), 11.0% succinoyl groups ($DS_{succinoyl}$ = 0.28), and a viscosity of 3.0 mPa·s, measured as a 2.0 % by weight solution of the HPMCAS in 0.43 wt.% aqueous NaOH.

HPMCAS used in Example 4

A mixture of the HPMCAS as used in Example 2 and another HPMCAS was used. The other HPMCAS had 23.0 % methoxyl groups ($DS_{methoxyl}$ = 1.89), 7.3 % hydroxypropoxyl groups ($MS_{hydroxypropoxyl}$ = 0.25), 9.8 % acetyl groups ($DS_{acetyl}$ = 0.58), 10.8 % succinoyl groups ($DS_{succinoyl}$ = 0.27), and a viscosity of 2.9 mPa·s, measured as a 2.0 % by weight solution of the HPMCAS in 0.43 wt.% aqueous NaOH.

Example 1

To produce an aqueous HPMCAS dispersion, HPMCAS and surfactant were added to water recirculating through a Drais DCP-12 Advantis media mill (1.0 mm ceramic media, 0.5 mm screen size). The mill speed was initially set at 1400 - 1500 rpm and then reduced as necessary down to about 1100 -1400 rpm to control the mill outlet temperature.
HPMCAS and surfactant were pre-blended at a weight ratio to provide a percentage of surfactant based on HPMCAS, as listed in Table 1 below. The pre-blend of HPMCAS and surfactant was loaded in portions to water recirculating through the mill. Addition continued until a total solids loading of about 30 % was achieved, based on the total weight of the composition. The percentage of HPMCAS, based on the total weight of the composition, was calculated from the measured solids content and the given weight ratio between HPMCAS and the surfactant. Following the addition of all solids, milling continued for about 3 hours until the final particle size was obtained. During the milling process, mild heat treatment was applied by adjusting the mill speed and the setpoint for the chiller system used to provide cooling to the mill jacket, tank and heat exchangers. The maximum temperature during milling was 39 - 41 °C.
The particle size distribution of the freshly produced aqueous HPMCAS dispersion was determined by laser diffraction particle size analysis as described further above. The results are listed in Table 1 below.
The produced aqueous HPMCAS dispersion was spray-dried with a mini spray dryer Büchi B290 under stirring (30 rpm with magnetic stirrer). The air inlet temperature into the spay-dryer was 175 °C; the outlet temperature of the moist air was 86 °C.
The resulting water-redispersible HPMCAS polymer powder was re-dispersed in water at the same concentration as listed in Table 1 below. The particle size distribution was determined by laser diffraction particle size analysis as described further above. The results are listed in Table 1 below.

Example 2

To produce an aqueous HPMCAS dispersion, the same mill as in Example 1 was used. The mill speed was initially set at 1300 - 1400 rpm and then reduced as necessary down to about 1100 rpm to control the mill outlet temperature.

HPMCAS and surfactant were pre-blended at a weight ratio to provide a percentage of surfactant, based on HPMCAS, as listed in Table 1 below. The pre-blend of HPMCAS and surfactant was loaded in portions to water recirculating through the mill. Co-surfactant was loaded in portions throughout to provide a percentage of co-surfactant based on HPMCAS as listed in Table 1 below. The co-surfactant "DSS 50%" as listed in Table 1 below was a 50 wt.-% solution of dioctyl sodium sulfosuccinate in polyethylene glycol 400, NF. The weight percent co-surfactant, based on HPMCAS, as listed in Table 1 below refers to the total weight of the 50% dioctyl sodium sulfosuccinate solution. Following the addition of all solids, milling continued for until the final particle size was obtained. The temperature during milling was 43 - 47 °C for 2¾ hours, for the remaining time the milling temperature was 35- 40°C. The total milling time was about 4.25 h. The particle size distribution of the freshly produced aqueous HPMCAS dispersion was determined as in Example 1.

The aqueous HPMCAS dispersion was diluted with deionized water to a total solids content of 25 wt. %. The diluted HPMCAS dispersion was spray-dried as described in Example 1 to produce a water-redispersible HPMCAS polymer power.

The resulting water-redispersible HPMCAS polymer powder was re-dispersed in water at the same concentration as listed in Table 1 below. The particle size distribution was determined by laser diffraction particle size analysis as described further above. The results are listed in Table 1 below.

Example 3

To produce an aqueous HPMCAS dispersion, the same mill as in Example 1 was used. The mill speed was initially set at 1300 - 1400 rpm and then reduced as necessary down to about 1100 rpm to control the mill outlet temperature.

HPMCAS and surfactant were pre-blended at a weight ratio to provide a percentage of surfactant, based on HPMCAS, as listed in Table 1 below. The pre-blend of HPMCAS and surfactant was loaded in portions to water recirculating through the mill. Co-surfactant was loaded in portions throughout to provide a percentage of co-surfactant based on HPMCAS as listed in Table 1 below. The co-surfactant was the same as in Example 2. Addition continued and mild heat treatment was applied during the subsequent milling as described in Example 1. The temperature during milling was most of the time from 30 to 40 °C, but during about 50 min. the temperature was more than 40 °C, the maximum being about 46 °C. The total milling time was about 2 hours. The particle size distribution of the freshly produced aqueous HPMCAS dispersion was determined as in Example 1.

The produced aqueous HPMCAS dispersion was diluted and spray-dried as described in Example 2 to produce a water-redispersible HPMCAS polymer power.

The resulting water-redispersible HPMCAS polymer powder was re-dispersed in water at the same concentration as listed in Table 1 below. The particle size distribution was determined by laser diffraction particle size analysis as described further above. The results are listed in Table 1 below.

Example 4

The aqueous HPMCAS dispersion was produced as in Example 3 except that the amount of surfactant and the HPMCAS concentration was as listed in Table 1 below and the temperature during milling was 43 - 47 °C for about 4 hours. The total milling time was about 4.25 hours. The particle size distribution of the freshly produced aqueous HPMCAS dispersion was determined as in Example 1.

The produced aqueous HPMCAS dispersion was diluted with deionized water to a total solids content of 30 wt. %. The diluted HPMCAS dispersion was spray-dried as described in Example 1 to produce a water-redispersible HPMCAS polymer power.

The resulting water-redispersible HPMCAS polymer powder was re-dispersed in water at the same concentration as listed in Table 1 below. The particle size distribution was determined by laser diffraction particle size analysis as described further above. The results are listed in Table 1 below.

The HPMCAS polymer powers of Examples 1 - 4 could be re-dispersed in water at the same concentration as in the original dispersion from which the water-redispersible HPMCAS polymer powers were prepared. The HPMCAS polymer powers could simply be added to water at room temperature and shaken for a short time period, e.g., for 1 minute, to disperse the HPMCAS powder in water. A stable dispersion resulted. Even after storage for several days no particle agglomeration and no sediment was observed.

Table 1

| (Comparative) Example | HPMCAS Dispersion before spray-drying | | | | | | | | | | Water-redispersible HPMCAS polymer power, particle size (μm) after re-dispersion in water | | | | | |
|---|---|---|---|---|---|---|---|---|---|---|---|---|---|---|---|---|
| | HPMCAS, based on total [%] | Wt.% Surfactant based on HPMCAS | Wt.% Co-surfactant, based on HPMCAS | Viscosity at 20 °C [mPa*s] | Particle size in freshly prepared HPMCAS dispersion (μm) | | | | | | | | | | | |
| | | | | | Mean | Dn10 | Dn25 | Dn50 | Dn75 | Dn90 | Mean | Dn10 | Dn25 | Dn50 | Dn75 | Dn90 |
| 1 | 28.5 | 4.8% Sodium Stearate | -- | 239 | 0.5 | 0.3 | 0.4 | 0.5 | 0.6 | 0.9 | 0.5 | 0.3 | 0.3 | 0.4 | 0.6 | 0.8 |
| 2 | 28.6 | 4.0% Sodium Stearate | 1.0% DSS 50% | 196 | 2.4 | 0.6 | 1.0 | 1.8 | 3.3 | 5.0 | 3.0 | 0.6 | 1.0 | 1.9 | 3.7 | 6.3 |
| 3 | 28.3 | 3.0 % Sodium Stearate | 1.0% DSS 50% | NA | 2.8 | 0.7 | 1.2 | 2.1 | 3.6 | 5.5 | 2.9 | 0.7 | 1.1 | 2.0 | 3.6 | 5.6 |
| 4 | 38.1 | 4.0% Sodium Stearate | 1.0% DSS 50% | NA | 2.3 | 0.6 | 1.0 | 1.7 | 3.2 | 4.9 | 2.7 | 0.8 | 1.2 | 2.2 | 3.7 | 5.4 |
| NA: not assessed | | | | | | | | | | | | | | | | |

Example 5: Preparation of Capsules

i) Preparation of Capsule Shells from HPMCAS dispersion

Immediately after preparation of the HPMCAS dispersion according to Example 1, before-spray-drying, the HPMCAS dispersion was cooled to 10 °C; 20 % triethyl citrate (TEC), based on the weight of HPMCAS, was added drop wise to the dispersion. Molding pins were pre-heated to a temperature of 70 °C and then dipped into the HPMCAS dispersion having a temperature of 10 °C. The pins were then withdrawn from the HPMCAS dispersion and a film was formed on the molding pins. The films on the molding pins were dried in a drying chamber having a temperature of 80 °C for 120 min. Capsule bodies resulted after drying.

ii) Preparation of Capsule Shells from water-redispersible HPMCAS polymer powder

Water was added to the water-redispersible HPMCAS polymer powder of Example 1. The amount of water was chosen to provide a HPMCAS concentration of 28.5 wt. %. This is the same HPMCAS concentration as in the dispersion from which the water-redispersible polymer HPMCAS powder of Example 1 had been produced. The addition of water was followed by shaking for 1 min. to disperse the HPMCAS powder in water. Addition of water and simply shaking for this short time period resulted in a stable dispersion. Even after storage for several days no particle agglomeration and no sediment was observed.

The HPMCAS dispersion was cooled to 10 °C; 20 % triethyl citrate (TEC), based on the weight of HPMCAS, was added drop wise to the dispersion. Molding pins were pre-heated and dipped into the HPMCAS dispersion and capsule bodies were produced as described above under section i) above. The same quality of capsule bodies was achieved as in section i) above where the capsules were prepared directly from the dispersion instead of from the water redispersed HPMCAS powder.

Comparative Example A

Micronized HPMCAS was used that is commercially available as Shin-Etsu AQOAT, grade micronized AS-MF. It has the specifications 21.0 - 25.0 % methoxyl content, 5.0 - 9.0 % hydroxypropoxyl content, 7.0 - 11.0 % acetyl content, 10.0 - 14.0 % succinoyl content, viscosity of 2.4 - 3.6 mPa·s, average particle size not more than 10 $\mu$m and 90 % cumulation not more than 20 $\mu$m. The particle sizes are measured by Shin-Etsu laser diffraction method.

An aqueous dispersion was produced as recommended on page 7 of the brochure "Hypromellose Acetate Succinate Shin-Etsu AQOAT, Enteric Coating Agent and as disclosed in U.S. Patent No. 5,837,291; Comparative Example 1. The aqueous dispersion contained 7.0 wt. % micronized Shin-Etsu AQOAT (AS-MF), 1.96 wt. % triethyl citrate, 2.1 wt. % talc, 0.21 wt. % sodium lauryl sulfate and 88.73 wt. % water.

When letting the dispersion (120 mL) stand in a graduated glass cylinder for two days, solid sediment formed already after 1h; i.e. the dispersion was not stable for an extended time period although it only contained 7.0 wt. % micronized Shin-Etsu AQOAT (AS-MF).

| | Comparative Example A | Example 3 | Example 4 |
|---|---|---|---|
| Storage time [h] | Sediment [mL] | Sediment [mL] | Sediment [mL] |
| 0 | No sediment | No sediment | No sediment |
| 1 | 4 | No sediment | No sediment |
| 2 | 5 | No sediment | No sediment |
| 3 | 7 | No sediment | No sediment |
| 4 | 9 | No sediment | No sediment |
| 5 | 9 | No sediment | No sediment |
| 24 | 15 | No sediment | No sediment |
| 48 | 15 | No sediment | No sediment |

**Claims**

1.  A water-redispersible polymer powder, wherein
    the polymer is at least one esterified cellulose ether comprising (i) groups of the formula -C(O) - R - COOA or (ii) a combination of aliphatic monovalent acyl groups and groups of the formula -C(O) - R - COOA, wherein R is a divalent aliphatic or aromatic hydrocarbon group and A is hydrogen or a cation, and
    the polymer powder comprises from 0.05 to 20 percent of at least one salt of a fatty acid, based on the weight of the esterified cellulose ether(s).

2.  The water-redispersible polymer powder of claim 1 comprising from 0.5 to 10 percent of an ammonium, alkali metal or alkaline earth metal salt of a saturated or unsaturated fatty acid, based on the weight of the esterified cellulose ether.

3.  The water-redispersible polymer powder of claim 1 or claim 2 wherein the salt of a fatty acid is an ammonium, alkali metal or alkaline earth metal salt of stearic acid or oleic acid.

4.  The water-redispersible polymer powder of any one of claims 1 to 3 wherein the degree of neutralization of the groups - C(O) - R - COOA is not more than 0.4.

5.  The water-redispersible polymer powder of any one of claims 1 to 4 wherein the aliphatic monovalent acyl groups in the esterified cellulose ether are acetyl, propionyl or butyryl groups, and the groups of the formula -C(O) - R - COOA are - C(O) - $CH_2$ - $CH_2$ - COOA, - C(O) - CH = CH - COOA, or - C(O) - $C_6H_4$ - COOA groups.

6.  The water-redispersible polymer powder of any one of claims 1 to 5 wherein the esterified cellulose ether is hydroxypropyl methyl cellulose acetate succinate.

7.  The water-redispersible polymer powder of any one of claims 1 to 6 wherein the median particle size, Dn50, of the powder particles is up to 5 micrometers, such median particle size, Dn50, being the size at which 50 number percent of the powder particles have a smaller equivalent diameter and 50 number percent have a larger equivalent diameter.

8.  The water-redispersible polymer powder of claim 7 wherein the median particle size, Dn50, of the powder particles is up to 4 micrometers.

9.  The water-redispersible polymer powder of any one of claims 1 to 8 wherein Dn90 of the powder particles is up to 12 micrometers, Dn90 being the diameter where 90 number percent of the powder particles have a smaller equivalent diameter and the other 10 number percent have a larger equivalent diameter.

10. A process for producing the water-redispersible polymer powder of any one of claims 1 to 9 comprising the steps of grinding, in the presence of an aqueous diluent, at least one esterified cellulose ether comprising (i) groups of the formula -C(O) - R - COOA or (ii) a combination of aliphatic monovalent acyl groups and groups of the formula -C(O) - R - COOA, wherein R is a divalent aliphatic or aromatic hydrocarbon group and A is hydrogen or a cation,
    blending from 0.05 to 20 percent of at least one salt of a fatty acid and optionally one or more adjuvants with the esterified cellulose ether before, during or after the grinding of the esterified cellulose ether, the percentage of the fatty acid salt being based on the weight of the esterified cellulose ether, and
    subjecting the produced aqueous composition to spray-drying.

11. A process for producing the water-redispersible polymer powder of any one of claims 1 to 9 comprising the steps of melting a) at least one esterified cellulose ether comprising
    (i) groups of the formula -C(O) - R - COOA or (ii) a combination of aliphatic monovalent acyl groups and groups of the formula -C(O) - R - COOA, wherein R is a divalent aliphatic or aromatic hydrocarbon group and A is hydrogen or a cation, and
    emulsifying the molten esterified cellulose ether in b) an aqueous diluent,
    adding c) from 0.05 to 20 percent of a salt of a fatty acid, based on the weight of the esterified cellulose ether, and optionally d) one or more adjuvants before, during or after the step of emulsifying the molten esterified cellulose ether in the aqueous diluent,
    cooling the emulsion to form an aqueous dispersion and
    subjecting the produced aqueous composition to spray-drying.

12. A process for producing the water-redispersible polymer powder of any one of claims 1 to 9 comprising the steps of

- providing an aqueous composition which comprises

a) dispersed particles of at least one esterified cellulose ether comprising (i) groups of the formula -C(O) - R - COOA or (ii) a combination of aliphatic monovalent acyl groups and groups of the formula -C(O) - R - COOA, wherein R is a divalent aliphatic or aromatic hydrocarbon group and A is hydrogen or a cation, and
b) from 0.05 to 20 percent of at least one salt of a fatty acid, based on the weight of the dispersed esterified cellulose ether, and

- subjecting the aqueous composition to spray-drying.

13. The process of any one of claims 10 to 12 wherein the median particle size, Dn50, of the dispersed particles is up to 5 micrometers, such median particle size, Dn50, being the size at which 50 number percent of the dispersed particles have a smaller equivalent diameter and 50 number percent have a larger equivalent diameter.

14. A process for coating a dosage form comprising the steps of

- dispersing the water-redispersible polymer powder of any one of claims 1 to 9 in water,
- adding a film forming aid and optional adjuvants to form an aqueous coating composition and
- coating a dosage form with the aqueous coating composition.

15. A process for producing capsule shells comprising the steps of

- dispersing the water-redispersible polymer powder of any one of claims 1 to 9 in water,
- adding a film forming aid and optional adjuvants to form an aqueous composition,
- pre-heating molding pins to a temperature higher than that of the aqueous composition,
- dipping the pre-heated molding pins into the aqueous composition,
- forming a film on said molding pins by withdrawing said pins from said aqueous composition, and
- drying the film on the molding pins.

**Patentansprüche**

1. Wasserredispergierbares Polymerpulver, wobei das Polymer wenigstens ein veresterter Celluloseether ist, der (i) Gruppen der Formel -C(O)-R-COOA oder (ii) eine Kombination von aliphatischen monovalenten Acylgruppen und Gruppen der Formel -C(O)-R-COOA enthält, wobei R eine divalente aliphatische oder aromatische Kohlenwasserstoffgruppe ist und A Wasserstoff oder ein Kation ist, und das Polymerpulver 0,05 bis 20 Gew.-% wenigstens eines Salzes einer Fettsäure, bezogen auf das Gewicht der veresterten Celluloseether, enthält.

2. Wasserredispergierbares Polymerpulver nach Anspruch 1, das 0,5 bis 10 Gew.-% eines Ammonium-, Alkalimetall- oder Erdalkalimetallsalzes einer gesättigten oder ungesättigten Fettsäure, bezogen auf das Gewicht des veresterten Celluloseethers, enthält.

3. Wasserredispergierbares Polymerpulver nach Anspruch 1 oder 2, wobei das Salz einer Fettsäure ein Ammonium-, Alkali- oder Erdalkalimetallsalz von Stearinsäure oder Ölsäure ist.

4. Wasserredispergierbares Polymerpulver nach einem der Ansprüche 1 bis 3, wobei der Neutralisationsgrad der Gruppen -C(O)-R-COOA nicht mehr als 0,4 beträgt.

5. Wasserredispergierbares Polymerpulver nach einem der Ansprüche 1 bis 4, wobei die aliphatischen monovalenten Acylgruppen in dem veresterten Celluloseether Acetyl-, Propionyl- oder Butyrylgruppen sind und die Gruppen der Formel -C(O)-R-COOA gleich -C(O)-CH$_2$-CH$_2$-COOA-, -C(O)-CH=CH-COOA- oder -C(O)-C$_6$H$_4$-COOA-Gruppen sind.

6. Wasserredispergierbares Polymerpulver nach einem der Ansprüche 1 bis 5, wobei der veresterte Celluloseether Hydroxypropylmethylcelluloseacetatsuccinat ist.

7. Wasserredispergierbares Polymerpulver nach einem der Ansprüche 1 bis 6, wobei die mittlere Teilchengröße Dn50 der Polymerteilchen bis zu 5 Mikrometer beträgt, wobei eine solche mittlere Teilchengröße Dn50 die Größe ist, bei der 50 Zahlenprozent der Polymerteilchen einen kleineren Äquivalentdurchmesser und 50 Zahlenprozent einen größeren Äquivalentdurchmesser aufweisen.

8. Wasserredispergierbares Polymerpulver nach Anspruch 7, wobei die mittlere Teilchengröße Dn50 der Pulverteilchen bis zu 4 Mikrometer beträgt.

9. Wasserredispergierbares Polymerpulver nach einem der Ansprüche 1 bis 8, wobei Dn90 der Pulverteilchen bis zu 12 Mikrometer beträgt, wobei Dn90 der Durchmesser ist, bei dem 90 Zahlenprozent der Pulverteilchen einen kleineren Äquivalentdurchmesser und die anderen 10 Zahlenprozent einen größeren Äquivalentdurchmesser haben.

10. Verfahren zur Herstellung des wasserredispergierbaren Polymerpulvers nach einem der Ansprüche 1 bis 9, umfassend die Schritte von Mahlen in Gegenwart eines wässrigen Verdünnungsmittels wenigstens eines veresterten Celluloseethers, der (i) Gruppen der Formel -C(O)-R-COOA oder (ii) eine Kombination von aliphatischen monovalenten Acylgruppen und Gruppen der Formel -C(O)-R-COOA enthält, wobei R eine divalente aliphatische oder aromatische Kohlenwasserstoffgruppe ist und A Wasserstoff oder ein Kation ist,
Mischen von 0,05 bis 20 Prozent wenigstens eines Salzes einer Fettsäure und optional eines oder mehrerer Hilfsmittel mit dem veresterten Celluloseether vor, während oder nach dem Mahlen des veresterten Celluloseethers, wobei die Prozentangabe bezüglich des Fettsäuresalzes sich auf das Gewicht des veresterten Celluloseethers bezieht, und Unterziehen der hergestellten wässrigen Zusammensetzung einer Sprühtrocknung.

11. Verfahren zur Herstellung des wasserredispergierbaren Polymerpulvers nach einem der Ansprüche 1 bis 9, umfassend die Schritte von Schmelzen von a) wenigstens einem veresterten Celluloseethers, der (i) Gruppen der Formel -C(O)-R-COOA oder (ii) eine Kombination von aliphatischen monovalenten Acylgruppen und Gruppen der Formel -C(O)-R-COOA enthält, wobei R eine divalente aliphatische oder aromatische Kohlenwasserstoffgruppe ist und A Wasserstoff oder ein Kation ist, und
Emulgieren des geschmolzenen veresterten Celluloseethers in b) in einem wässrigen Verdünnungsmittel, Zugeben c) von 0,05 bis 20 Prozent eines Salzes einer Fettsäure, bezogen auf das Gewicht des veresterten Celluloseethers, und optional d) eines oder mehrerer Hilfsstoffe vor, während oder nach dem Emulgieren des geschmolzenen veresterten Celluloseethers in einem wässrigen Verdünnungsmittel,
Kühlen der Emulsion, um eine wässrige Dispersion zu bilden, und Unterziehen der wässrigen Zusammensetzung einer Sprühtrocknung.

12. Verfahren zur Herstellung des wasserredispergierbaren Polymerpulvers nach einem der Ansprüche 1 bis 9, umfassend die Schritte von

    - Bereitstellen einer wässrigen Zusammensetzung, die

        a) dispergierte Teilchen wenigstens eines veresterten Celluloseethers, der (i) Gruppen der Formel -C(O)-R-COOA oder (ii) eine Kombination von aliphatischen monovalenten Acylgruppen und Gruppen der Formel -C(O)-R-COOA enthält, wobei R eine divalente aliphatische oder aromatische Kohlenwasserstoffgruppe ist und A Wasserstoff oder ein Kation ist, und
        b) von 0,05 bis 20 Prozent wenigstens eines Salzes einer Fettsäure, bezogen auf das Gewicht des dispergierten veresterten Celluloseethers,
        enthält, und

    - Unterziehen der wässrigen Zusammensetzung einer Sprühtrocknung.

13. Verfahren nach einem der Ansprüche 10 bis 12, wobei die mittlere Teilchengröße Dn50 der dispergierten Teilchen bis zu 5 Mikrometer beträgt und eine solche mittlere Teilchengröße Dn50 die Größe ist, bei der 50 Zahlenprozent der dispergierten Teilchen einen kleineren Äquivalentdurchmesser und 50 Zahlenprozent einen größeren Äquivalentdurchmesser aufweisen.

14. Verfahren zur Beschichtung einer Dosierform, umfassend die Schritte von

    - Dispergieren des wasserredispergierbaren Polymerpulvers nach einem der Ansprüche 1 bis 9 in Wasser,
    - Zugeben einer Filmbildungshilfe und optionaler Hilfsstoffe, um eine wässrige Beschichtungszusammensetzung zu bilden, und

- Beschichten einer Dosierform mit der wässrigen Beschichtungszusammensetzung.

**15.** Verfahren zur Herstellung von Kapselschalen, umfassend die Schritte von

- Dispergieren des wasserredispergierbaren Polymerpulvers nach einem der Ansprüche 1 bis 9 in Wasser,
- Zugeben einer Filmbildungshilfe und optionaler Hilfsstoffe, um eine wässrige Zusammensetzung zu bilden,
- Vorerwärmen von Formgebungsstiften auf eine Temperatur, die höher ist als die der wässrigen Zusammensetzung,
- Tauchen der vorerwärmten Formgebungsstifte in die wässrige Zusammensetzung,
- Ausbilden eines Films auf diesen Formgebungsstiften durch Herausziehen der Stifte aus dieser wässrigen Zusammensetzung und
- Trocknen des Films auf den Formgebungsstiften.

**Revendications**

**1.** Poudre polymère redispersable dans l'eau, dans laquelle

- le polymère est au moins un éther de cellulose estérifié portant (i) des groupes de formule -C(O)-R-COOA ou (ii) une combinaison de groupes acyle monovalents aliphatiques et de groupes de formule -C(O)-R-COOA, R symbolisant un groupe hydrocarboné divalent aliphatique ou aromatique, et A représentant un atome d'hydrogène ou un cation,
- et la poudre polymère comprend au moins un sel d'acide gras en une proportion, rapportée au poids d'éther(s) de cellulose estérifié(s), valant de 0,05 % à 20 %.

**2.** Poudre polymère redispersable dans l'eau conforme à la revendication 1, qui comprend un sel d'ammonium, de métal alcalin ou de métal alcalino-terreux d'un acide gras saturé ou insaturé, en une proportion, rapportée au poids d'éther de cellulose estérifié, valant de 0,5 % à 10 %.

**3.** Poudre polymère redispersable dans l'eau conforme à la revendication 1 ou la revendication 2, dans laquelle le sel d'acide gras est un sel d'ammonium, de métal alcalin ou de métal alcalino-terreux d'acide stéarique ou d'acide oléique.

**4.** Poudre polymère redispersable dans l'eau conforme à l'une quelconque des revendications 1 à 3, dans laquelle le degré de neutralisation des groupes de formule -C(O)-R-COOA ne dépasse pas 0,4.

**5.** Poudre polymère redispersable dans l'eau conforme à l'une quelconque des revendications 1 à 4, dans laquelle les groupes acyle monovalents aliphatiques portés par l'éther de cellulose estérifié sont des groupes acétyle, propionyle ou butyryle, et les groupes de formule -C(O)-R-COOA sont des groupes de formule -C(O)-CH$_2$-CH$_2$-COOA, -C(O)-CH=CH-COOA ou -C(O)-C$_6$H$_4$-COOA.

**6.** Poudre polymère redispersable dans l'eau conforme à l'une quelconque des revendications 1 à 5, dans laquelle l'éther de cellulose estérifié est de l'acétate-succinate d'hydroxypropyl-méthyl-cellulose.

**7.** Poudre polymère redispersable dans l'eau conforme à l'une quelconque des revendications 1 à 6, dans laquelle la dimension de particule médiane, Dn50, des particules de poudre vaut jusqu'à 5 $\mu$m, cette dimension de particule médiane, Dn50, étant la dimension à laquelle le diamètre équivalent de 50 % en nombre des particules de poudre est inférieur et le diamètre équivalent de 50 % en nombre de ces particules est supérieur.

**8.** Poudre polymère redispersable dans l'eau conforme à la revendication 7, dans laquelle la dimension de particule médiane, Dn50, des particules de poudre vaut jusqu'à 4 $\mu$m.

**9.** Poudre polymère redispersable dans l'eau conforme à l'une quelconque des revendications 1 à 8, dans laquelle la dimension Dn90 des particules de poudre vaut jusqu'à 12 $\mu$m, Dn90 représentant le diamètre auquel le diamètre équivalent de 90 % en nombre des particules de poudre est inférieur et le diamètre équivalent des 10 % en nombre restants de ces particules est supérieur.

**10.** Procédé en vue de produire une poudre polymère redispersable dans l'eau conforme à l'une quelconque des revendications 1 à 9, qui comprend les étapes consistant à

- broyer, en présence d'un diluant aqueux, au moins un éther de cellulose estérifié portant (i) des groupes de formule -C(O)-R-COOA ou (ii) une combinaison de groupes acyle monovalents aliphatiques et de groupes de formule -C(O)-R-COOA, R symbolisant un groupe hydrocarboné divalent aliphatique ou aromatique, et A représentant un atome d'hydrogène ou un cation,
- combiner au moins un sel d'acide gras, en une proportion valant de 0,05 % à 20 %, et, en option, un ou plusieurs adjuvant(s) avec l'éther de cellulose estérifié, avant, pendant ou après le broyage de l'éther de cellulose estérifié, le pourcentage de sel d'acide gras étant rapporté au poids d'éther de cellulose estérifié,
- et exposer la composition aqueuse ainsi obtenue à un séchage par atomisation.

11. Procédé en vue de produire une poudre polymère redispersable dans l'eau conforme à l'une quelconque des revendications 1 à 9, qui comprend les étapes consistant à

- faire fondre (a) au moins un éther de cellulose estérifié portant (i) des groupes de formule -C(O)-R-COOA ou (ii) une combinaison de groupes acyle monovalents aliphatiques et de groupes de formule -C(O)-R-COOA, R symbolisant un groupe hydrocarboné divalent aliphatique ou aromatique, et A représentant un atome d'hydrogène ou un cation,
- et émulsifier l'éther de cellulose estérifié à l'état fondu dans (b) un diluant aqueux,
- ajouter (c) un sel d'acide gras en une proportion, rapportée au poids d'éther de cellulose estérifié, valant de 0,05 % à 20 %, et, en option, (d) un ou plusieurs adjuvant(s), avant, pendant ou après l'étape consistant à émulsifier l'éther de cellulose estérifié à l'état fondu dans le diluant aqueux,
- faire refroidir l'émulsion de manière à former une dispersion aqueuse,
- et exposer la composition aqueuse ainsi obtenue à un séchage par atomisation.

12. Procédé en vue de produire une poudre polymère redispersable dans l'eau conforme à l'une quelconque des revendications 1 à 9, qui comprend les étapes consistant à

- se procurer une composition aqueuse qui comporte

(a) des particules dispersées d'au moins un éther de cellulose estérifié portant (i) des groupes de formule -C(O)-R-COOA ou (ii) une combinaison de groupes acyle monovalents aliphatiques et de groupes de formule -C(O)-R-COOA, R symbolisant un groupe hydrocarboné divalent aliphatique ou aromatique, et A représentant un atome d'hydrogène ou un cation,
(b) et au moins un sel d'acide gras en une proportion, rapportée au poids d'éther de cellulose estérifié à l'état dispersé, valant de 0,05 % à 20 %,

- et exposer la composition aqueuse à un séchage par atomisation.

13. Procédé conforme à l'une quelconque des revendications 10 à 12, dans lequel la dimension de particule médiane, Dn50, des particules dispersées vaut jusqu'à 5 μm, cette dimension de particule médiane, Dn50, étant la dimension à laquelle le diamètre équivalent de 50 % en nombre des particules dispersées est inférieur et le diamètre équivalent de 50 % en nombre de ces particules est supérieur.

14. Procédé en vue d'enrober une forme galénique, qui comprend les étapes consistant à

- disperser dans de l'eau une poudre polymère redispersable dans l'eau conforme à l'une quelconque des revendications 1 à 9,
- ajouter un auxiliaire filmogène et des adjuvants optionnels, de manière à former une composition d'enrobage aqueuse,
- et enrober une forme galénique avec la composition d'enrobage aqueuse ainsi obtenue.

15. Procédé en vue de produire des enveloppes de capsules, qui comprend les étapes consistant à

- disperser dans de l'eau une poudre polymère redispersable dans l'eau conforme à l'une quelconque des revendications 1 à 9,
- ajouter un auxiliaire filmogène et des adjuvants optionnels, de manière à former une composition aqueuse,
- préchauffer des broches de moulage à une température supérieure à celle de la composition aqueuse,

- tremper les broches de moulage ainsi préchauffées dans la composition aqueuse,
- former un film sur lesdites broches de moulage en retirant lesdites broches de ladite composition aqueuse,
- et faire sécher le film ainsi obtenu sur les broches de moulage.

## REFERENCES CITED IN THE DESCRIPTION

**Patent documents cited in the description**

- US 4226981 A
- US 4365060 A
- JP 7070203 A
- EP 0648487 A
- US 4462839 A
- US 5837291 A
- US 5539021 A
- US 7763676 B
- WO 2013164122 A
- WO 2013164121 A

**Non-patent literature cited in the description**

- Hypromellose Acetate Succinate. *United States Pharmacopeia and National Formulary, NF 29,* 1548-1550
- Hypromellose. *United States Pharmacopeia and National Formulary, USP,* vol. 35, 3467-3469
- Hypromellose Acetate Succinate. *United States Pharmacopia and National Formulary, NF 29,* 1548-1550
- Hypromellose Acetate Succinate. *United States Pharmacopia and National Formulary,* January 1999, 1548-1550
- Hypromellose Acetate Succinate. *United States Pharmacopia and National Formulary,* 1548-1550